# EUROPEAN PATENT SPECIFICATION

(11) **EP 3 709 958 B1**
(45) Date of publication and mention of the grant of the patent: **06.12.2023**
(21) Application number: 18800963.3
(22) Date of filing: 15.11.2018
(51) Int. Cl.: A61K 8/41, A61Q 1/02, A61Q 17/04, A61K 8/81, A61Q 19/08, A61K 8/06

(54) **COSMETIC OR DERMATOLOGICAL COMPOSITION COMPRISING A MEROCYANINE AND AN ACRYLIC POLYMER**
KOSMETISCHE ODER DERMATOLOGISCHE ZUSAMMENSETZUNG MIT EINEM MEROCYANIN UND EINEM ACRYLPOLYMER
COMPOSITION COSMETIQUE OU DERMATOLOGIQUE COMPRENANT UNE MEROCYANINE ET UN POLYMERE ACRYLIQUE

(30) Priority: 15.11.2017 FR 1760733
(43) Date of publication of application: 23.09.2020
(73) Proprietor: L'OREAL, 75008 Paris (FR)
(72) Inventor: SAFOUANE, Mahassine, 94152 Chevilly La Rue (FR); LUCET-LEVANNIER, Karine, 93400 Saint-Ouen (FR); ABEYSEKERA, Nideka, 94152 Chevilly La Rue (FR)
(74) Representative: L'Oreal
(86) International application number: PCT/EP2018/081471
(87) International publication number: WO 2019/096956

(56) References cited:
- EP-A1- 3 235 839
- FR-A1- 3 001 136
- US-A- 4 128 635

## Description

The present invention relates to a cosmetic or dermatological composition in the emulsion form, comprising in a physiologically acceptable support:
a) at least one aqueous phase and
b) at least one oily phase and
c) at least one merocyanine compound of formula (1) that will be defined below in detail and
d) at least one particular acrylic polymer.

Also described is a non-therapeutic cosmetic process for caring for and/or making up a keratin material, comprising the application, to the surface of said keratin material, of at least one composition according to the invention as defined above.

Also described is a non-therapeutic cosmetic process for limiting the darkening of the skin and/or improving the colour and/or uniformity of the complexion, comprising the application, to the surface of the keratin material, of at least one composition as defined previously.

Also described is a non-therapeutic cosmetic process for preventing and/or treating the signs of ageing of a keratin material, comprising the application, to the surface of the keratin material, of at least one composition as defined previously.

It is known that radiation with wavelengths of between 280 nm and 400 nm makes possible tanning of the human epidermis and that radiation with wavelengths of between 280 and 320 nm, known under the name of UV-B rays, harms the development of a natural tan. Exposure is also liable to induce impairment of the biomechanical properties of the epidermis, which is reflected by the appearance of wrinkles, leading to premature ageing of the skin.

It is also known that UV-A rays with wavelengths of between 320 and 400 nm penetrate more deeply into the skin than UV-B rays. UV-A rays cause immediate and persistent browning of the skin. Daily exposure to UVA rays, even of short duration, under normal conditions can result in damage to the collagen fibres and the elastin, which is reflected by a modification to the microrelief of the skin, the appearance of wrinkles and uneven pigmentation (liver spots, heterogeneity of the complexion).

Protection against UVA and UVB radiation is therefore necessary. An effective photoprotective product must protect against both UVA and UVB radiation.

Many photoprotective compositions have been proposed to date to overcome the effects induced by UVA and/or UVB radiation. They generally contain organic UV screening agents and/or inorganic UV screening agents, which function according to their own chemical nature and according to their own properties by absorption, reflection or scattering of the UV radiation. They generally contain mixtures of liposoluble organic screening agents and/or of water-soluble UV-screening agents combined with metal oxide pigments such as titanium dioxide or zinc oxide.

Many cosmetic compositions intended to limit darkening of the skin, and to improve the colour and uniformity of the complexion have been proposed to date. It is well known in the field of suntan products that such compositions can be obtained by using UV-screening agents, and in particular UVB-screening agents. Some compositions may also contain UVA-screening agents. This screening system must cover UVB protection for the purpose of limiting and controlling the neo-synthesis of melanin promoting overall pigmentation, but must also cover UVA protection in order to limit and control the oxidation of the already existing melanin resulting in darkening of the colour of the skin.

However, no composition contains a particular combination of UV-screening agents that would be specially suitable for photoprotection of the skin and particularly for an improvement in the quality of the skin both in terms of the colour and in terms of its mechanical elasticity properties.

Advantageously, this improvement is particularly visible on skin that is already pigmented, for the purpose of not increasing either the pigmentary melanin load or the structure of the melanin already present within the skin.

In fact, the majority of organic UV-screening agents are constituted of aromatic compounds which absorb in the wavelength range between 280 and 370 nm. In addition to their solar radiation-screening capacity, the desired photoprotective compounds must also have good cosmetic properties, good solubility in the usual solvents and in particular in fatty substances such as oils or fats, and also good photostability alone or in combination with other UV-screening agents. They must also be colourless or at least have a colour that is cosmetically acceptable for consumers.

One of the main drawbacks known to date of these compositions is that these screening systems have insufficient efficiency against UV radiation and particularly against long UVA radiation with wavelengths above 370 nm with the aim of controlling photoinduced pigmentation and the evolution thereof by means of a system which screens out UV radiation over the whole of the UV spectrum.

Among all the compounds that have been recommended for this purpose, an advantageous family of UV-screening agents which is constituted of carbonated merocyanine derivatives has been proposed, which is described in patent US 4 195 999, application WO 2004/006878 and document IP COM Journal 4 (4), 16 No.IPCOM000011179D published on 04/03/2004. These compounds have very good screening properties in the long UVA radiation range, but have poorly satisfactory solubility in the usual solvents and in particular in fatty substances such as oils, and an unsatisfactory photostability for some merocyanines.

With the aim of searching for other merocyanines which have better solubility in the usual solvents and better photostability, application PCT/EP2012/064195 has proposed merocyanines comprising polar groups constituted of hydroxyl and ether functions, which show good long UVA-screening efficiency.

Anti-sun compositions are fairly often provided in the form of an emulsion of oil-in-water type (that is to say, a cosmetically acceptable support constituted of a continuous aqueous dispersing phase and of a non-continuous oily dispersed phase) or of the water-in-oil type (that is to say, a cosmetically acceptable support constituted of a continuous oily dispersing phase and of a non-continuous aqueous dispersed phase) which comprises, at various concentrations, one or more conventional lipophilic and/or hydrophilic organic screening agents which are capable of selectively absorbing harmful UV rays, these screening agents (and their amounts) being selected as a function of the desired sun protection factor.

The applicant has noted, during the course of its research, that some of the merocyanines identified in application PCT/EP2012/064195 can bring about the destabilization of the compositions in the presence of certain emulsifiers.

There remains therefore the need to select other families of emulsifiers which guarantee a better stability of the compositions which comprise merocyanines comprising polar groups constituted of hydroxyl and ether functions.

The applicant has discovered, surprisingly, that the emulsifying systems containing at least one polymer as defined below make it possible to achieve this objective. Thus, in accordance with one of the subjects of the present invention, a cosmetic or dermatological composition in emulsion form is now provided which comprises, in a physiologically acceptable support:
a) at least one aqueous phase and
b) at least one oily phase and
c) at least one merocyanine compound of formula (1) that will be defined below in detail and
d) at least one polymer defined below.

Also described is a non-therapeutic cosmetic process for caring for and/or making up a keratin material, which consists in applying, to the surface of said keratin material, at least one composition according to the invention as defined above.

Also described is a non-therapeutic cosmetic process for limiting the darkening of the skin and improving the colour and uniformity of the complexion, comprising at least the application, to the surface of the keratin material, of at least one composition as defined previously.

Also described is a non-therapeutic cosmetic process for treating the ageing of a keratin material, comprising at least the application, to the surface of the keratin material, of at least one composition as defined previously.

Other characteristics, aspects and advantages of the invention will become apparent on reading the detailed description that follows.

The term "human keratin materials" is intended to mean the skin (of the body, face and around the eyes), hair, eyelashes, eyebrows, body hair, nails, lips or mucous membranes.

The term "physiologically acceptable" is intended to mean compatible with the skin and/or its integuments, which has a pleasant colour, odour and feel, and which does not cause any unacceptable discomfort (stinging, tautness or redness) liable to discourage the consumer from using this composition.

The term "between X and Y" is intended to mean the range of values also including the limits X and Y.

According to the invention, the term "preventing" or "prevention" is intended to mean reducing the risk of occurrence or slowing down the occurrence of a given phenomenon, namely, according to the present invention, the signs of ageing of a keratin material.

The term "emulsion" is intended to mean any macroscopically homogeneous, kinetically stable composition comprising at least two mutually immiscible phases; one being the dispersing continuous phase and the other being dispersed in the said continuous phase in the form of droplets. The two phases are kinetically stabilized by at least one emulsifying system generally comprising at least one emulsifying surfactant.

A distinction is made between emulsions of oil-in-water type, termed "direct", constituted of a continuous aqueous dispersing phase and of a non-continuous oily dispersed phase, and emulsions of water-in-oil type, termed "inverse", constituted of a continuous oily dispersing phase and of a non-continuous aqueous dispersed phase. Multiple emulsions, such as water-in-oil-in-water or oil-in-water-in-oil, also exist.

### MEROCYANINES

According to the present invention, the merocyanine compounds in accordance with the invention correspond to formula (1) below, and also the E/E- or E/Z- geometrical isomer forms thereof: 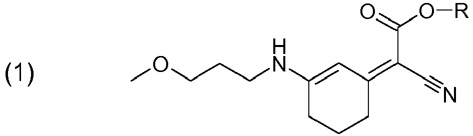 in which
R is a C₁-C₂₂ alkyl group, a C₂-C₂₂ alkenyl group, a C₂-C₂₂ alkynyl group, a C₃-C₂₂ cycloalkyl group or a C₃-C₂₂ cycloalkenyl group, said groups possibly being interrupted with one or more O.

The merocyanine compounds of the invention may be in the E/E- or E/Z- geometrical isomer forms thereof.

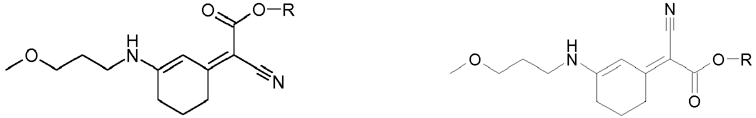

The preferential compounds of formula (1) are those wherein:
R is a C₁-C₂₂ alkyl, which may be interrupted with one or more O.

Among the compounds of formula (1), use will more particularly be made of those chosen from the following compounds and also the E/E- or E/Z- geometrical isomer forms thereof:

| | | | |
|---|---|---|---|
| 1 | 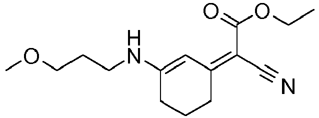 ethyl (2Z)-cyano{3-[(3-methoxypropyl)amino]cyclohex-2-en-1-ylidene}ethanoate | 4 | 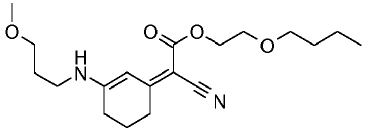 2-butoxyethyl (2Z)-cyano{3-[(3-methoxypropyl)amino]cyclohex-2-en-1-ylidene}ethanoate |
| 2 | 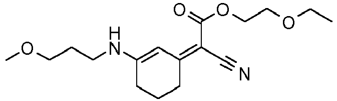 2-ethoxyethyl (2Z)-cyano{3-[(3-methoxypropyl)amino]cyclohex-2-en-1-ylidene}ethanoate | 5 | 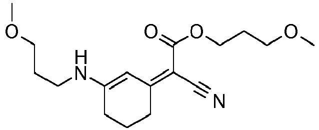 3-methoxypropyl (2Z)-cyano{3-[(3-methoxypropyl)amino]cyclohex-2-en-1-ylidene}ethanoate |
| 3 | 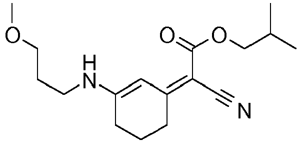 2-methylpropyl (2Z)-cyano{3-[(3-methoxypropyl)amino]cyclohex-2-en-1-ylidene}ethanoate | 6 | 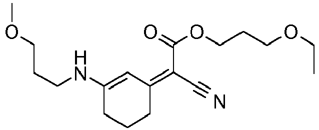 3-ethoxypropyl (2Z)-cyano{3-[(3-methoxypropyl)amino]cyclohex-2-en-1-ylidene}ethanoate |

According to one more particularly preferred mode of the invention, use will be made of the compound 2-ethoxyethyl (2Z)-cyano{3-[(3-methoxypropyl)amino]cyclohex-2-en-1-ylidene}ethanoate (2) in its E/Z geometrical configuration having the following structure: 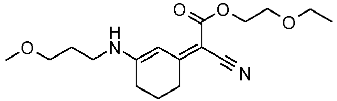 and/or in its E/E geometrical configuration having the following structure:

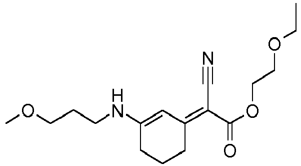

The merocyanines of formula (1) according to the invention are preferably present in the compositions according to the invention in a concentration ranging from 0.1% to 10% by weight, and preferentially from 0.2% to 5% by weight relative to the total weight of the composition.

The compounds of formula (I) may be prepared according to the protocols described in patent application WO 2007/071582, in IP.com Journal (2009), 9(5A), 29-30 IPCOM000182396D under the title "Process for producing 3-amino-2-cyclohexan-1-ylidene compounds" and in US-A-4,749,643 in col. 13, line 66 - col. 14, line 57 and the references cited in this regard.

### ACRYLIC POLYMER

According to the invention, the polymer d) comprises monomeric units of formulae (A) and (B): 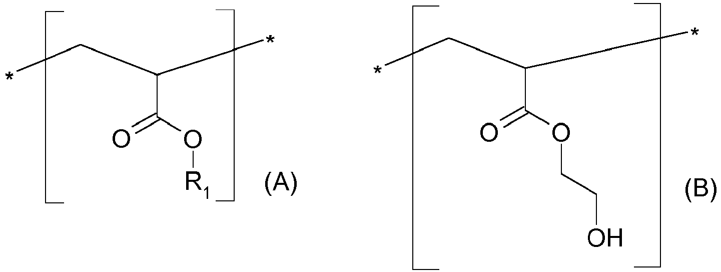 in which:
R₁, independently of one another, is chosen from alkyl or alkylene radicals;
   and
at least 60% by weight of the R₁ groups are radicals chosen from stearyl and behenyl radicals, the percentage by weight relating to the sum of all the R₁ groups present in the polymer;
   and
the weight ratio of the sum of all the hydroxyethyl acrylate units to the sum of all the acrylate units bearing the R₁ group ranges from 1: 30 to 1: 1,
and the sum of the total of units A and B is at least 95% by weight of the total weight of the polymer.

Preferably, R₁ is constituted of alkyl radicals, preferably of C₁₆-C₂₂ alkyl radicals, and more preferentially of stearyl (C₁₈) radicals or of behenyl (C₂₂) radicals.

Preferably, at least 70% by weight of the R₁ groups are stearyl or behenyl radicals, preferentially at least 80% by weight, more preferentially at least 90% by weight. According to one preferred embodiment, all the R₁ groups are behenyl radicals. According to another preferred embodiment, all the R₁ groups are stearyl radicals.

Preferably, said weight ratio ranges from 1:15 to 1:1, preferentially ranges from 1:10 to 1:4.

Advantageously, the polymer units present in the polymer are constituted of the units (A) and (B) previously described.

The polymer has a number-average molecular weight Mn ranging from 2000 to 9000 g/mol, preferably ranging from 5000 to 9000 g/mol. The number-average molecular weight can be measured by the gel permeation chromatography method, for example according to the method described in the example hereinbelow.

Preferably, the polymer has a melting point ranging from 40°C to 70°C, and preferentially ranging from 45°C to 67°C. The melting point is measured by differential scanning calorimetry (DSC), for example according to the method described in the example hereinbelow.

According to a first embodiment, when the polymer is such that at least 60% by weight of the R₁ groups are stearyl radicals, then the polymer preferably has a melting point ranging from 40 to 60°C, and preferentially ranging from 45 to 55°C. According to a second embodiment, when the polymer is such that at least 60% by weight of the R₁ groups are behenyl radicals, then the polymer has a melting point ranging from 60°C to 70°C, and preferentially ranging from 63°C to 67°C.

The polymer used according to the invention can be prepared by polymerization of a monomer of formula
CH₂=CH-COO-R₁, R₁ having the meaning previously described, and of 2-hydroxyethyl acrylate.

The polymerization may be performed according to known methods, such as solution polymerization or emulsion polymerization.

The polymerization is, for example, described in document US 2007/0264204.

The polymer d) can be present in the composition according to the invention in a content of active material ranging from 0.05 to 10% by weight, preferably ranging from 0.1 to 5% by weight and better still ranging from 0.2 to 2% by weight, relative to the total weight of the composition.

### OILY PHASE

The compositions in accordance with the invention comprise at least one oily phase.

For the purposes of the invention, the term "oily phase" is intended to mean a phase comprising at least one oil and all of the liposoluble and lipophilic ingredients and fatty substances used for the formulation of the compositions of the invention.

The term "oil" is intended to mean any fatty substance that is in liquid form at ambient temperature (20-25°C) and atmospheric pressure (760 mmHg).

An oil that is suitable for use in the invention may be volatile or non-volatile.

An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" is intended to mean an oil comprising at least one silicon atom, and especially at least one Si-O group.

The term "hydrocarbon-based oil" is intended to mean an oil mainly containing hydrogen and carbon atoms.

The term "fluoro oil" is intended to mean an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent(s), at least one volatile or non-volatile hydrocarbon-based oil and/or one volatile and/or non-volatile silicone oil.

For the purposes of the invention, the term "volatile oil" refers to an oil that is capable of evaporating on contact with the skin or the keratin fibre in less than one hour, at ambient temperature and atmospheric pressure. The volatile oil(s) of the invention are volatile cosmetic oils which are liquid at ambient temperature and which have a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is intended to mean an oil that remains on the skin or the keratin fibre at ambient temperature and atmospheric pressure for at least several hours, and that especially has a vapour pressure of less than 10⁻³ mmHg (0.13 Pa).

### Hydrocarbon-based oils

Mention may in particular be made, as non-volatile hydrocarbon-based oils which can be used according to the invention, of:
(i) hydrocarbon-based oils of plant origin, such as glyceride triesters, which are generally triesters of fatty acids and of glycerol, the fatty acids of which can have varied chain lengths from C₄ to C₂₄, it being possible for these chains to be saturated or unsaturated and linear or branched; these oils are in particular wheatgerm oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, sesame oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stearineries Dubois or those sold under the names Miglyol 8100, 8120 and 818^{®} by Dynamit Nobel;
(ii) synthetic ethers having from 10 to 40 carbon atoms;
(iii) linear or branched hydrocarbons of mineral or synthetic origin, such as petroleum jelly, polydecenes, hydrogenated polyisobutene, such as Parleam, squalane and mixtures thereof;
(iv) synthetic esters, such as the oils of formula RCOOR' in which R represents a linear or branched fatty acid residue comprising from 1 to 40 carbon atoms and R' represents a hydrocarbon-based chain that is especially branched, containing from 1 to 40 carbon atoms, with the proviso that R + R' ≥ 10, for instance purcellin oil (cetostearyl octanoate), isopropyl myristate, isopropyl palmitate, C₁₂-C₁₅ alkyl benzoate, such as the product sold under the trade name Finsolv TN^{®} or Witconol TN^{®} by the company Witco or Tegosoft TN^{®} by the company Evonik Goldschmidt, 2-ethylphenyl benzoate, such as the commercial product sold under the name X-Tend 226^{®} by the company ISP, isopropyl lanolate, hexyl laurate, diisopropyl adipate, isononyl isononanoate, oleyl erucate, 2-ethylhexyl palmitate, isostearyl isostearate, diisopropyl sebacate such as the product sold under the name Dub Dis by the company Stearineries Dubois, alcohol or polyalcohol octanoates, decanoates or ricinoleates, such as propylene glycol dioctanoate; hydroxylated esters, such as isostearyl lactate, diisostearyl malate; and pentaerythritol esters; citrates or tartrates, such as linear C₁₂-C₁₃ dialkyl tartrates, such as those sold under the name Cosmacol ETI^{®} by the company Enichem Augusta Industriale, and also linear C₁₄-C₁₅ dialkyl tartrates such as those sold under the name Cosmacol ETL^{®} by the same company; acetates.
(v) fatty alcohols which are liquid at ambient temperature and which have a branched and/or unsaturated carbon chain comprising from 12 to 26 carbon atoms, such as octyldodecanol, isostearyl alcohol, oleyl alcohol, 2-hexyldecanol, 2-butyloctanol or 2-undecylpentadecanol;
(vi) higher fatty acids, such as oleic acid, linoleic acid or linolenic acid;
(vii) carbonates, such as dicaprylyl carbonate, such as the product sold under the name Cetiol CC^{®} by the company Cognis;
(viii) fatty amides, such as isopropyl N-lauroyl sarcosinate, such as the product sold under the trade name Eldew SL205^{®} from the company Ajinomoto, and mixtures thereof.

Among the non-volatile hydrocarbon-based oils that may be used according to the invention, preference will be given more particularly to glyceride triesters and in particular to caprylic/capric acid triglycerides, synthetic esters and in particular isononyl isononanoate, oleyl erucate, C₁₂-C₁₅ alkyl benzoate, 2-ethylphenyl benzoate and fatty alcohols, in particular octyldodecanol.

As volatile hydrocarbon-based oils that may be used according to the invention, mention may be made in particular of hydrocarbon-based oils containing from 8 to 16 carbon atoms and in particular of branched C₈-C₁₆ alkanes, such as C₈-C₁₆ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched C₈-C₁₆ esters, isohexyl neopentanoate, and mixtures thereof.

Mention may also be made of the alkanes described in the Cognis patent applications WO 2007/068 371 or WO 2008/155 059 (mixtures of distinct alkanes differing by at least one carbon). These alkanes are obtained from fatty alcohols, which are themselves obtained from coconut or palm oil. Mention may be made of the mixtures of n-undecane (C₁₁) and n-tridecane (C₁₃) obtained in Examples 1 and 2 of patent application WO 2008/155 059 from the company Cognis. Mention may also be made of n-dodecane (C₁₂) and n-tetradecane (C₁₄) sold by Sasol under the respective references Parafol 12-97 and Parafol 14-97^{®}, and also mixtures thereof.

Other volatile hydrocarbon-based oils, for instance petroleum distillates, in particular those sold under the name Shell Solt^{®} by the company Shell, may also be used. According to one embodiment, the volatile solvent is chosen from volatile hydrocarbon-based oils having from 8 to 16 carbon atoms and mixtures thereof.

### b) Silicone oils

The non-volatile silicone oils can be chosen in particular from non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Volatile silicone oils that may be mentioned, for example, include volatile linear or cyclic silicone oils, in particular those with a viscosity ≤ 8 centistokes (8×10⁻⁶ m²/s) and in particular containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I): 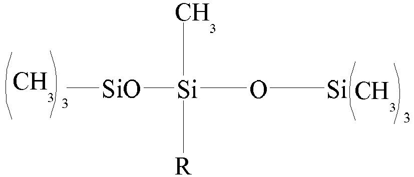 where R represents an alkyl group comprising from 2 to 4 carbon atoms, one or more hydrogen atoms of which may be replaced with a fluorine or chlorine atom.

Among the oils of general formula (I), mention may be made of:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

### Fluoro oils

Use may also be made of volatile fluoro oils, such as nonafluoromethoxybutane, decafluoropentane, tetradecafluorohexane, dodecafluoropentane, and mixtures thereof.

An oily phase according to the invention may also comprise other fatty substances, mixed with or dissolved in the oil.

Another fatty substance that may be present in the oily phase may be, for example:
- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

Preferentially, the overall oily phase, including all the lipophilic substances of the composition capable of being dissolved in this same phase, represents from 5% to 95% by weight and preferentially from 10% to 80% by weight, relative to the total weight of the composition.

### AQUEOUS PHASE

The compositions according to the invention comprise at least one aqueous phase.

The aqueous phase contains water and optionally other water-soluble or water-miscible organic solvents.

An aqueous phase that is suitable for use in the invention may comprise, for example, a water chosen from a natural spring water, such as water from La Roche-Posay, water from Vittel or waters from Vichy, or a floral water.

The water-soluble or water-miscible solvents that are suitable for use in the invention comprise short-chain monoalcohols, for example C₁-C₄ monoalcohols, such as ethanol or isopropanol; diols or polyols, such as ethylene glycol, 1,2-propylene glycol, 1,3-butylene glycol, pentylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.

According to a preferred embodiment, use may more particularly be made of ethanol, propylene glycol, glycerol, and mixtures thereof.

According to one particular form of the invention, the overall aqueous phase, including all the hydrophilic substances of the composition capable of being dissolved in this same phase, represents from 5% to 95% by weight and preferentially from 10% to 80% by weight, relative to the total weight of the composition.

### ADDITIVES

### a) Additional UV-screening agents:

The compositions according to the invention may also contain one or more additional UV-screening agents chosen from hydrophilic, lipophilic or insoluble organic UV-screening agents and/or one or more mineral pigments. It will preferentially be constituted of at least one hydrophilic, lipophilic or insoluble organic UV-screening agent.

The term "hydrophilic UV-screening agent" is intended to mean any cosmetic or dermatological organic or mineral compound for screening out UV radiation, which can be fully dissolved in molecular form in a liquid aqueous phase or which can be dissolved in colloidal form (for example in micellar form) in a liquid aqueous phase.

The term "lipophilic screening agent" is intended to mean any cosmetic or dermatological organic or mineral compound for screening out UV radiation, which can be fully dissolved in molecular form in a liquid fatty phase or which can be dissolved in colloidal form (for example in micellar form) in a liquid fatty phase.

The term "insoluble UV-screening agent" is intended to mean any cosmetic or dermatological organic or inorganic compound for screening out UV radiation which has a solubility in water of less than 0.5% by weight and a solubility of less than 0.5% by weight in the majority of organic solvents such as liquid paraffin, fatty alcohol benzoates and fatty acid triglycerides, for example Miglyol 812^{®} sold by the company Dynamit Nobel. This solubility, determined at 70°C, is defined as the amount of product in solution in the solvent at equilibrium with an excess of solid in suspension after returning to ambient temperature. It may be readily evaluated in the laboratory.

The additional organic UV-screening agents are chosen in particular from cinnamic compounds; anthranilate compounds; salicylic compounds; dibenzoylmethane compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole derivatives; imidazoline compounds; bis-benzazolyl compounds, as described in patents EP 669 323 and US 2 463 264; p-aminobenzoic (PABA) compounds; methylenebis(hydroxyphenylbenzotriazole) compounds, as described in patent applications US 5 237 071, US 5 166 355, GB 2 303 549, DE 197 26 184 and EP 893 119; benzoxazole compounds, as described in patent applications EP 0 832 642, EP 1 027 883, EP 1 300 137 and DE 101 62 844; screening polymers and screening silicones, such as those described in particular in patent application WO 93/04665; α-alkylstyrene-based dimers, such as those described in patent application DE 198 55 649; 4,4-diarylbutadiene compounds, as described in patent applications EP 0 967 200, DE 197 46 654, DE 197 55 649, EP-A-1 008 586, EP 1 133 980 and EP 133 981, and mixtures thereof.

As examples of organic photoprotective agents, mention may be made of those denoted hereinbelow under their INCI name:

### Cinnamic compounds:

Ethylhexyl methoxycinnamate sold in particular under the trade name Parsol MCX^{®} by DSM Nutritional Products,
Isopropyl methoxycinnamate,
Isoamyl p-methoxycinnamate sold under the trade name Neo Heliopan E 1000^{®} by Symrise,
DEA methoxycinnamate,
Diisopropyl methylcinnamate,
Glyceryl ethylhexanoate dimethoxycinnamate.

### para-Aminobenzoic compounds:

PABA,
Ethyl PABA,
Ethyl dihydroxypropyl PABA,
Ethylhexyl dimethyl PABA, sold in particular under the name Escalol 507^{®} by ISP, Glyceryl PABA,
PEG-25 PABA, sold under the name Uvinul P 25^{®} by BASF.

### Salicylic compounds:

Homosalate, sold under the name Eusolex HMS^{®} by Rona/EM Industries,
Ethylhexyl salicylate, sold under the name Neo Heliopan OS^{®} by Symrise,
Dipropylene glycol salicylate, sold under the name Dipsal^{®} by Scher,
TEA salicylate, sold under the name Neo Heliopan TS^{®} by Symrise.

### β,β-Diphenylacrylate compounds:

Octocrylene, sold in particular under the trade name Uvinul N 539^{®} by BASF,
Etocrylene, sold in particular under the trade name Uvinul N 35^{®} by BASF.

### Benzophenone compounds:

Benzophenone-1 sold under the trade name Uvinul 400^{®} by BASF,
Benzophenone-2, sold under the trade name Uvinul D 50^{®} by BASF,
Benzophenone-3 or Oxybenzone, sold under the trade name Uvinul M 40^{®} by BASF,
Benzophenone-4, sold under the trade name Uvinul MS 40^{®} by BASF,
Benzophenone-5,
Benzophenone-6 sold under the trade name Helisorb 11^{®} by Norquay,
Benzophenone-8, sold under the trade name Spectra-Sorb UV-24^{®} by American Cyanamid,
Benzophenone-9, sold under the trade name Uvinul DS 49^{®} by BASF,
Benzophenone-12,
n-Hexyl 2-(4-diethylamino-2-hydroxybenzoyl)benzoate, sold under the trade name Uvinul A Plus^{®} or, as a mixture with octyl methoxycinnamate, under the trade name Uvinul A Plus B^{®} by the company BASF,
1,1'-(1,4-Piperazinediyl)bis[1-[2-[4-(diethylamino)-2-hydroxybenzoyl]phenyl]methanone] (CAS 919803-06-8), such as described in patent application WO 2007/071 584; this compound advantageously being used in micronized form (average size of 0.02 to 2 µm), which may be obtained, for example, according to the micronization process described in patent applications GB-A-2 303 549 and EP-A-893 119, and in particular in the form of an aqueous dispersion.

### Benzylidenecamphor compounds:

3-Benzylidenecamphor, manufactured under the name Mexoryl SD^{®} by Chimex, 4-Methylbenzylidenecamphor, sold under the name Eusolex 6300^{®} by Merck,
Benzylidenecamphorsulfonic acid, manufactured under the name Mexoryl SL^{®} by Chimex,
Camphor benzalkonium methosulfate, manufactured under the name Mexoryl SO^{®} by Chimex,
Terephthalylidenedicamphorsulfonic acid, manufactured under the name Mexoryl SX^{®} by Chimex,
Polyacrylamidomethylbenzylidenecamphor, manufactured under the name Mexoryl SW^{®} by Chimex.

### Phenylbenzimidazole compounds:

Phenylbenzimidazolesulfonic acid, sold in particular under the trade name Eusolex 232^{®} by Merck.

### Bis-benzazolyl compounds

Disodium phenyl dibenzimidazole tetrasulfonate, sold under the trade name Neo Heliopan AP^{®} by Haarmann and Reimer.

### Phenylbenzotriazole compounds:

Drometrizole trisiloxane, sold under the name Silatrizole^{®} by Rhodia Chimie.

### Methylenebis(hydroxyphenylbenzotriazole) compounds:

Methylenebis(benzotriazolyl)tetramethylbutylphenol, in particular in solid form, such as the product sold under the trade name Mixxim BB/100^{®} by Fairmount Chemical, or in the form of an aqueous dispersion of micronized particles with an average particle size ranging from 0.01 to 5 µm, more preferentially from 0.01 to 2 µm and more particularly from 0.020 to 2 µm, with at least one alkylpolyglycoside surfactant having the structure CₙH₂ₙ₊₁O(C₆H₁₀O₅)ₓH, in which n is an integer from 8 to 16 and x is the mean degree of polymerization of the (C₆H₁₀O₅) unit and ranges from 1.4 to 1.6, as described in patent GB-A-2 303 549, sold in particular under the trade name Tinosorb M^{®} by the company BASF, or in the form of an aqueous dispersion of micronized particles with an average particle size ranging from 0.02 to 2 µm, more preferentially from 0.01 to 1.5 µm and more particularly from 0.02 to 1 µm, in the presence of at least one polyglyceryl mono(C₈-C₂₀)alkyl ester with a degree of glycerol polymerization of at least 5, such as the aqueous dispersions described in patent application WO 2009/063 392.

### Triazine compounds:

- Bis-ethylhexyloxyphenol methoxyphenyl triazine, sold under the trade name Tinosorb S^{®} by BASF,
- Ethylhexyl triazone, sold in particular under the trade name Uvinul T150^{®} by BASF,
- Diethylhexyl butamido triazone, sold under the trade name Uvasorb HEB^{®} by Sigma 3V,
- 2,4,6-tris(dineopentyl 4'-aminobenzalmalonate)-s-triazine,
- 2,4,6-tris(diisobutyl 4'-aminobenzalmalonate)-s-triazine,
- 2,4-bis(n-butyl 4'-aminobenzoate)-6-(aminopropyltrisiloxane)-s-triazine,
- 2,4-bis(dineopentyl 4'-aminobenzalmalonate)-6-(n-butyl 4'-aminobenzoate)-s-triazine,
- symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 µm), which may be obtained, for example, according to the micronization process described in patent applications GB-A-2 303 549 and EP-A-893 119, and in particular in aqueous dispersion form,
- Silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

### Anthranilic compounds:

Menthyl anthranilate, sold under the trade name Neo Heliopan MA^{®} by Symrise.

### Imidazoline compounds:

Ethylhexyl dimethoxybenzylidene dioxoimidazoline propionate.

### Benzalmalonate compounds:

Polyorganosiloxane comprising benzalmalonate functions, such as Polysilicone-15, sold under the trade name Parsol SLX^{®} by Hoffmann-LaRoche.

### 4,4-Diarylbutadiene compounds:

1,1-Dicarboxy(2,2'-dimethylpropyl)-4,4-diphenylbutadiene.

### Benzoxazole compounds:

2,4-Bis[5-(1-dimethylpropyl)benzoxazol-2-yl(4-phenyl)imino]-6-(2-ethylhexyl)imino-1,3,5-triazine, sold under the name Uvasorb K2A^{®} by Sigma 3V.

The preferential organic screening agents are chosen from:
Ethylhexyl methoxycinnamate
Ethylhexyl salicylate,
Homosalate,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
Methylenebis(benzotriazolyl)tetramethylbutylphenol,
Bis-ethylhexyloxyphenol methoxyphenyl triazine,
Ethylhexyl triazone
Diethylhexyl butamidotriazone,
2,4,6-tris(dineopentyl 4'-aminobenzalmalonate)-s-triazine,
2,4,6-Tris(diisobutyl 4'-aminobenzalmalonate)-s-triazine,
2,4-Bis(n-butyl 4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
2,4,6-Tris(diphenyl)triazine,
2,4,6-Tris(terphenyl)triazine,
Drometrizole trisiloxane,
Polysilicone-15,
1,1-Dicarboxy(2,2'-dimethylpropyl)-4,4-diphenylbutadiene,
2,4-bis[5-(1-Dimethylpropyl)benzoxazol-2-yl-(4-phenyl)imino]-6-(2-ethylhexyl)imino-1,3,5-triazine,
and mixtures thereof.

The particularly preferred organic screening agents are chosen from:
Ethylhexyl salicylate,
Homosalate,
Octocrylene,
n-Hexyl 2-(4-diethylamino-2-hydroxybenzoyl)benzoate,
Terephthalylidenedicamphorsulfonic acid,
Bis-ethylhexyloxyphenol methoxyphenyl triazine,
Ethylhexyl triazone
Diethylhexyl butamidotriazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole trisiloxane,
and mixtures thereof.

The inorganic UV-screening agents used in accordance with the present invention are metal oxide pigments. More preferentially, the inorganic UV-screening agents of the invention are metal oxide particles with a mean elementary particle size of less than or equal to 0.5 µm, more preferentially between 0.005 and 0.5 µm, even more preferentially between 0.01 and 0.2 µm, better still between 0.01 and 0.1 µm and more particularly between 0.015 and 0.05 µm.

They may be chosen in particular from titanium oxide, zinc oxide, iron oxide, zirconium oxide and cerium oxide, or mixtures thereof.

Such coated or non-coated metal oxide pigments are described in particular in Patent Application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides coated:
- with silica, such as the product Sunveil^{®} from the company Ikeda,
- with silica and iron oxide, such as the product Sunveil F^{®} from the company Ikeda,
- with silica and alumina, such as the products Microtitanium Dioxide MT 500 SA^{®} and Microtitanium Dioxide MT 100 SA^{®} from the company Tayca and Tioveil from the company Tioxide,
- with alumina, such as the products Tipaque TTO-55 (B)^{®} and Tipaque TTO-55 (A)^{®} from the company Ishihara and UVT 14/4 from the company Sachtleben Pigments,
- with alumina and aluminium stearate, such as the products Microtitanium Dioxide MT 100 T^{®}, MT 100 TX^{®}, MT 100 Z^{®} and MT-01^{®} from the company Tayca, the products Solaveil CT-10 W^{®} and Solaveil CT 100^{®} from the company Uniqema and the product Eusolex T-AVO^{®} from the company Merck,
- with silica, alumina and alginic acid, such as the product MT-100 AQ^{®} from the company Tayca,
- with alumina and aluminium laurate, such as the product Microtitanium Dioxide MT 100 S^{®} from the company Tayca,
- with iron oxide and iron stearate, such as the product Microtitanium Dioxide MT 100 F^{®} from the company Tayca,
- with zinc oxide and zinc stearate, such as the product BR 351^{®} from the company Tayca,
- with silica and alumina and treated with a silicone, such as the products Microtitanium Dioxide MT 600 SAS^{®}, Microtitanium Dioxide MT 500 SAS^{®} or Microtitanium Dioxide MT 100 SAS^{®} from the company Tayca,
- with silica, alumina and aluminium stearate and treated with a silicone, such as the product STT-30-DS^{®} from the company Titan Kogyo,
- with silica and treated with a silicone, such as the product UV-Titan X 195^{®} from the company Sachtleben Pigments,
- with alumina and treated with a silicone, such as the products Tipaque TTO-55 (S)^{®} from the company Ishihara or UV Titan M 262^{®} from the company Sachtleben Pigments,
- with triethanolamine, such as the product STT-65-S from the company Titan Kogyo,
- with stearic acid, such as the product Tipaque TTO-55 (C)^{®} from the company Ishihara,
- with sodium hexametaphosphate, such as the product Microtitanium Dioxide MT 150 W^{®} from the company Tayca,
- TiO₂ treated with octyltrimethylsilane, sold under the trade name T 805^{®} by the company Degussa Silices,
- TiO₂ treated with a polydimethylsiloxane, sold under the trade name 70250 Cardre UF TiO2SI3^{®} by the company Cardre,
- anatase/rutile TiO₂ treated with a polydimethylhydrogenosiloxane, sold under the trade name Microtitanium Dioxide USP Grade Hydrophobic^{®} by the company Color Techniques.

Mention may also be made of TiO₂ pigments doped with at least one transition metal such as iron, zinc or manganese and more particularly manganese. Preferably, said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG-3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of TiO₂ particles doped with manganese in capric/caprylic acid triglyceride in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50^{®} by the company Croda.

The uncoated titanium oxide pigments are sold, for example, by the company Tayca under the trade names Microtitanium Dioxide MT 500 B or Microtitanium Dioxide MT 600 B^{®}, by the company Degussa under the name P 25, by the company Wackherr under the name Transparent titanium oxide PW^{®}, by the company Miyoshi Kasei under the name UFTR^{®}, by the company Tomen under the name ITS^{®} and by the company Tioxide under the name Tioveil AQ^{®}.

The non-coated zinc oxide pigments are, for example:
- those sold under the Z-Cote name by the company Sunsmart;
- those sold under the name Nanox^{®} by the company Elementis;
- those sold under the name Nanogard WCD 2025^{®} by the company Nanophase Technologies.

The coated zinc oxide pigments are, for example:
- those sold under the name Zinc Oxide CS-5^{®} by the company Toshibi (ZnO coated with polymethylhydrogenosiloxane);
- those sold under the name Nanogard Zinc Oxide FN^{®} by the company Nanophase Technologies (as a 40% dispersion in Finsolv TN^{®}, C12-C15 alkyl benzoate);
- those sold under the name Daitopersion Zn-30^{®} and Daitopersion Zn-50^{®} by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO^{®} by the company Daikin (ZnO coated with perfluoroalkyl phosphate and copolymer based on perfluoroalkylethyl as a dispersion in cyclopentasiloxane);
- those sold under the name SPD-Z1^{®} by the company Shin-Etsu (ZnO coated with silicone-grafted acrylic polymer, dispersed in cyclodimethylsiloxane);
- those sold under the name Escalol Z100^{®} by the company ISP (alumina-treated ZnO dispersed in an ethylhexyl methoxycinnamate/PVP-hexadecene/methicone copolymer mixture);
- those sold under the name Fuji ZnO-SMS-10^{®} by the company Fuji Pigment (ZnO coated with silica and polymethylsilsesquioxane);
- those sold under the name Nanox Gel TN^{®} by the company Elementis (ZnO dispersed at a concentration of 55% in C₁₂-C₁₅ alkyl benzoate with hydroxystearic acid polycondensate).

The uncoated cerium oxide pigments may be, for example, those sold under the name Colloidal Cerium Oxide^{®} by the company Rhone-Poulenc.

The uncoated iron oxide pigments are sold, for example, by the company Arnaud under the names Nanogard WCD 2002^{®} (FE 45B^{®}), Nanogard Iron FE 45 BL AQ^{®}, Nanogard FE 45R AQ^{®} and Nanogard WCD 2006^{®} (FE 45R^{®}) or by the company Mitsubishi under the name TY-220^{®}.

The coated iron oxide pigments are sold, for example, by the company Arnaud under the names Nanogard WCD 2008 (FE 45B FN)^{®}, Nanogard WCD 2009^{®} (FE 45B 556^{®}), Nanogard FE 45 BL 345^{®} and Nanogard FE 45 BL^{®} or by the company BASF under the name Transparent Iron Oxide^{®}.

Mention may also be made of mixtures of metal oxides, especially of titanium dioxide and of cerium dioxide, including the equal-weight mixture of titanium dioxide and cerium dioxide coated with silica, sold by the company Ikeda under the name Sunveil A^{®}, and also the mixture of titanium dioxide and zinc dioxide coated with alumina, silica and silicone, such as the product M 2610 sold by the company Sachtleben Pigments, or coated with alumina, silica and glycerol, such as the product M 2110 sold by the company Sachtleben Pigments.

According to the invention, coated or uncoated titanium oxide pigments are particularly preferred.

The additional UV-screening agents according to the invention are preferably present in the compositions according to the invention in a content ranging from 0.1% to 45% by weight and in particular from 5% to 30% by weight relative to the total weight of the composition.

### b) Other additives:

The aqueous compositions in accordance with the present invention may also comprise conventional cosmetic adjuvants chosen in particular from organic solvents, ionic or non-ionic thickeners, softeners, humectants, opacifiers, stabilizers, emollients, silicones, antifoams, fragrances, preservatives, anionic, cationic, non-ionic, zwitterionic or amphoteric surfactants, active agents, fillers, polymers, propellants, basifying or acidifying agents or any other ingredient commonly used in the cosmetic and/or dermatological field.

Mention may be made, among organic solvents, of lower alcohols and polyols. These polyols may be chosen from glycols and glycol ethers, for instance ethylene glycol, propylene glycol, butylene glycol, dipropylene glycol or diethylene glycol.

Thickeners that may be mentioned include carboxyvinyl polymers such as the Carbopol products (carbomers) and the Pemulen products (acrylate/C₁₀-C₃₀-alkyl acrylate copolymer) (Pemulen TR1^{®} or Pemulen TR2^{®}); polyacrylamides, for instance the crosslinked copolymers sold under the names Sepigel 305^{®} (CTFA name: polyacrylamide/C₁₃₋₁₄ isoparaffin/Laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, such as the poly(2-acrylamido-2-methylpropanesulfonic acid) sold by the company Hoechst under the trade name Hostacerin AMPS^{®} (CTFA name: ammonium polyacryloyldimethyl taurate) or Simulgel 800^{®}, sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, such as Simulgel NS^{®} and Sepinov EMT 10^{®}, sold by the company SEPPIC; cellulose derivatives, such as hydroxyethylcellulose; polysaccharides and in particular gums, such as xanthan gum; water-soluble or water-dispersible silicone derivatives, such as acrylic silicones, polyether silicones and cationic silicones, and mixtures thereof.

Among the acidifying agents that may be mentioned, by way of example, are mineral or organic acids, for instance hydrochloric acid, orthophosphoric acid or sulfuric acid, carboxylic acids, for instance acetic acid, tartaric acid, citric acid and lactic acid, and sulfonic acids.

Among the basifying agents, examples that may be mentioned include aqueous ammonia, alkali metal carbonates, alkanolamines, such as mono-, di- and triethanolamines and derivatives thereof, sodium hydroxide or potassium hydroxide.

Preferably, the cosmetic composition comprises one or more basifying agents chosen from alkanolamines, in particular triethanolamine, and sodium hydroxide.

The compositions according to the invention can contain emulsifying surfactants suited to the type of emulsion.

For the O/W emulsions, examples of emulsifiers that may be mentioned include non-ionic emulsifiers such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG-100 stearate/glyceryl stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by Henkel under the respective names Plantaren 2000 and Plantaren 1200, cetostearyl glucoside, optionally as a mixture with cetostearyl alcohol, sold, for example, under the name Montanov 68 by SEPPIC, under the name Tegocare CG90 by Goldschmidt and under the name Emulgade KE3302 by Henkel, and also arachidyl glucoside, for example in the form of the mixture of arachidyl alcohol, behenyl alcohol and arachidyl glucoside, sold under the name Montanov 202 by SEPPIC. According to one particular embodiment of the invention, the mixture of the alkyl polyglucoside as defined above with the corresponding fatty alcohol can be in the form of a self-emulsifying composition, for example as described in the document WO-A-92/06778.

According to one particular form of the invention, use will likewise also be made of C₁₁-C₁₈ fatty acids such as undecanoic acid, lauric acid, such as the products sold under the trade name Acide Laurique 98^{®} by the company Stearinerie Dubois, or Edenor C12-99 MY^{®} by the company Emery Oleochemicals, palmitic acid, such as the products sold under the trade name Palmera A9516^{®} or Palmera A9816^{®} by the company KLK Oleo, or Acide Palmitique 95% - Pastilles by the company Stearinerie Dubois, or the stearic acid sold under the trade name Palmera B1802CG by the company KLK Oleo, Stearine TP 1200 Pastilles^{®} (Dub 50P^{®}) by the company Stearinerie Dubois, or Kortacid PH 05.02^{®} by the company Pacific Oleochemicals, or mixtures thereof, for example such as the mixture of stearic and palmitic acid sold under the trade name Dub Microlub 50^{®} by the company Stearinerie Dubois, and more particularly stearic acid. The fatty acids may be present in the compositions of the invention preferably at between 0.1% and 5%, and more particularly between 0.5% and 3%.

As emulsifying surfactants that may be used for the preparation of the W/O emulsions, examples that may be mentioned include sorbitan, glycerol or sugar alkyl esters or ethers; silicone surfactants, for instance dimethicone copolyols, such as the mixture of cyclomethicone and of dimethicone copolyol, sold under the name DC 5225 C by the company Dow Corning, and alkyldimethicone copolyols such as laurylmethicone copolyol sold under the name Dow Corning 5200 Formulation Aid by the company Dow Corning; cetyl dimethicone copolyol, such as the product sold under the name Abil EM 90R by the company Goldschmidt, and the mixture of cetyldimethicone copolyol, of polyglyceryl isostearate (4 mol) and of hexyl laurate, sold under the name Abil WE 09 by the company Goldschmidt. One or more coemulsifiers, which may be chosen advantageously from the group comprising polyol alkyl esters, may also be added thereto.

Polyol alkyl esters that may especially be mentioned include polyethylene glycol esters, for instance PEG-30 dipolyhydroxystearate, such as the product sold under the name Arlacel P135 by the company ICI.

Glycerol and/or sorbitan esters that may be mentioned include, for example, polyglyceryl isostearate, such as the product sold under the name Isolan GI 34 by Goldschmidt; sorbitan isostearate, such as the product sold under the name Arlacel 987 by ICI; sorbitan glyceryl isostearate, such as the product sold under the name Arlacel 986 by ICI, and mixtures thereof.

In the case of a direct emulsion, the pH of the composition in accordance with the invention is generally between 3 and 12 approximately, preferably between 5 and 11 approximately and even more particularly from 6 to 8.5.

Among the active agents for caring for keratin materials such as the skin, the lips, the scalp, the hair, the eyelashes or the nails, examples that may be mentioned include:
- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or for preventing their degradation;
- agents for stimulating fibroblast proliferation;
- agents for stimulating keratinocyte proliferation;
- muscle relaxants;
- refreshing agents;
- tensioning agents;
- matting agents;
- depigmenting agents;
- pro-pigmenting agents;
- keratolytic agents;
- desquamating agents;
- moisturizers;
- anti-inflammatory agents;
- antimicrobial agents;
- slimming agents;
- agents that act on the energy metabolism of cells;
- insect repellents;
- substance P or CGRP antagonists;
- hair-loss counteractants;
- antiwrinkle agents;
- antiageing agents.

Those skilled in the art will select said active agent(s) according to the effect desired on the skin, the hair, the eyelashes, the eyebrows or the nails.

Of course, those skilled in the art will take care to choose the abovementioned optional additional compound or compounds and/or their amounts so that the advantageous properties intrinsically attached to the compositions in accordance with the invention are not, or not substantially, detrimentally affected by the envisaged addition or additions.

### PRESENTATION FORMS

The compositions according to the invention can be prepared according to the techniques well known to those skilled in the art for producing emulsions, and in particular oil-in-water emulsions. They can be in the form of a cream, a milk or a gel-cream. They may optionally be in the form of a mousse or a spray.

Preferably, the compositions according to the invention are in the form of an oil-in-water emulsion.

The emulsification processes that may be used are of the paddle or propeller, rotor-stator and HPH type.

In order to obtain stable emulsions with a low content of polymer (oil/polymer ratio > 25), it is possible to prepare the dispersion in concentrated phase and then to dilute the dispersion with the remainder of the aqueous phase.

It is also possible, by means of an HPH (between 50 and 800 bar), to obtain stable dispersions with drop sizes that may be as low as 100 nm.

The aqueous phase of the emulsions may comprise a non-ionic vesicular dispersion prepared according to known processes (Bangham, Standish and Watkins, J. Mol. Biol. 13, 238 (1965), FR 2 315 991 and FR 2 416 008).

The compositions according to the invention find their application in a large number of treatments, in particular cosmetic treatments, for the skin, the lips and the hair, including the scalp, in particular for protecting and/or caring for the skin, the lips and/or the hair, and/or for making up the skin and/or the lips.

Another subject of the present invention is constituted of the use of the compositions according to the invention as defined above for the manufacture of products for the cosmetic treatment of the skin, the lips, the nails, the hair, the eyelashes, the eyebrows and/or the scalp, in particular care products, antisun products and makeup products.

The cosmetic compositions according to the invention may be used, for example, as makeup products.

Also described is a non-therapeutic cosmetic process for caring for and/or making up a keratin material, which consists in applying, to the surface of said keratin material, at least one composition according to the invention as defined above.

The cosmetic compositions according to the invention may be used, for example, as care products and/or antisun products for the face and/or body, with a liquid to semi-liquid consistency, such as milks, more or less smooth creams, gel-creams or pastes. They may optionally be packaged in aerosol form and may be in the form of a mousse or a spray.

The compositions according to the invention in the form of vaporizable fluid lotions in accordance with the invention are applied to the skin or hair in the form of fine particles by means of pressurizing devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged in aerosol form in accordance with the invention generally contain conventional propellants, for instance hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

### ASSEMBLY

According to another aspect, the invention also relates to a cosmetic assembly comprising:
i) a container delimiting one or more compartment(s), said container being closed by a closing member and optionally not being leaktight; and
ii) a makeup and/or care composition in accordance with the invention placed inside said compartment(s).

The container may be, for example, in the form of a jar or a box.

The closing member can be in the form of a lid comprising a cap mounted so as to be able to move by translation or by pivoting relative to the container housing said makeup and/or care composition(s).

The examples that follow serve to illustrate the invention without, however, being limiting in nature. In these examples, the amounts of the composition ingredients are given as weight percentages relative to the total weight of the composition.

### Example A1: Preparation of compound (1)

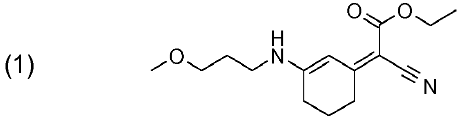

122.23 grams of 3-[(3-methoxypropyl)amino]-2-cyclohexen-1-one were alkylated with dimethyl sulfate or alternatively with diethyl sulfate and treated with 75.45 grams of ethyl cyanoacetate in approximately equimolar proportions in the presence of a base and optionally of a solvent. The following base/solvent combinations were used:

| **Example** | **Base** | **Solvent** |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction could be monitored for example by methods such as TLC, GC or HPLC. 162.30 grams of compound (14) were obtained in the form of a brown oil. After crystallization, the product was obtained in the form of yellowish crystals. Melting point: 92.7°C.

### Example A2: Preparation of compound (2)

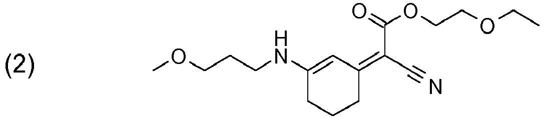

148.4 grams of 3-[(3-methoxypropyl)amino]-2-cyclohexen-1-one were alkylated with dimethyl sulfate or alternatively with diethyl sulfate and treated with 130.00 grams of 2-ethoxyethyl cyanoacetate in the presence of an organic base and of a solvent. The following base/solvent combinations were used:

| **Example** | **Base** | **Solvent** |
|---|---|---|
| Example A2.1 | DBU (1,8-diazabicyclo[5.4.0]undec-7-ene) | dimethylacetamide |
| Example A2.2 | triethylamine | isopropanol |
| Example A2.3 | 3-methoxypropylamine | isopropanol |
| Example A2.4 | N-methylmorpholine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | No solvent |

### Example of preparation of polymer 1:

Determination of the molecular weight by gel permeation chromatography (GPC): The sample is prepared by preparing a solution of the polymer at 10 mg/ml in tetrahydrofuran. The sample is placed in an oven at 54°C for 10 minutes and then in an oscillating shaker for 60 minutes to aid dissolution. After visual inspection, the sample appears to be totally dissolved in the solvent.

The sample prepared was analysed using two polypore 300×7.5 mm columns (manufactured by Agilent Technologies), a Waters 2695 chromatographic system, a tetrahydrofuran mobile phase and detection by refractive index. The sample was filtered through a 0.45 µm nylon filter, before being injected into the liquid chromatograph. The standards used for the calibration are the Easi Vial narrow polystyrene (PS) standards from Agilent Technologies.

Polystyrene standards ranging from 2 520 000 to 162 daltons were used for the calibration.

The system is equipped with a PSS SECcurity 1260 RI detector. The polystyrene calibration curve was used to determine the average molecular weight. The recording of the diagrams and the determination of the various molecular weights were performed by the Win GPC Unichrom 81 program.

Determination of the melting point by differential scanning calorimetry (or DSC): This method describes the general procedure for determining the melting point of polymers by differential scanning calorimetry. This method is based on the standards ASTM E791 and ASTM D 34182 and the DSC calibration is performed according to standard ASTM E 9672.

### Behenyl acrylate / 2-hydroxyethyl acrylate copolymer (Polymer 1):

In a 4-necked flask equipped with a side-blade mixer, an internal thermometer, two funnels, a reflux condenser, and an extension for two other necks, 175 g of behenyl acrylate, 25 g of 2-hydroxyethyl acrylate and 0.4 g of 2,2'-azobis(2-methylbutyronitrile) (Akzo Nobel) were added, over the course of 60 minutes at 80°C, to 40 g of isopropanol, with stirring, after having removed the oxygen from the system by means of a nitrogen flush for 20 minutes. The mixture was stirred at 80°C for 3 hours. The solvent was then eliminated by vacuum distillation, then 1 g of dilauryl peroxide was added and the reaction was continued for 60 minutes at 110°C. The step was repeated. The mixture was then cooled to 90°C, a stream of demineralized water was added and the mixture was then stirred. The water was removed by vacuum distillation.
Molecular weight: Mn = 7300 g/mol, Mw = 21 000, Mw/Mn = 2.8
Melting point: 65°C

### Example of preparation of polymer 2:

### Stearyl acrylate / 2-hydroxyethyl acrylate copolymer (Polymer 2):

In a 4-necked flask equipped with a side-blade mixer, an internal thermometer, two funnels, a reflux condenser, and an extension for two other necks, 155 g of behenyl acrylate, 45 g of 2-hydroxyethyl acrylate and 0.4 g of 2,2'-azobis(2-methylbutyronitrile) (Akzo Nobel) were added, over the course of 90 minutes at 80°C, to 50 g of isopropanol, with stirring, after having removed the oxygen from the system by means of a nitrogen flush for 20 minutes. The mixture was stirred at 80°C for 3 hours. The solvent was then eliminated by vacuum distillation, then 1 g of dilauryl peroxide was added and the reaction was continued for 60 minutes at 125°C. The step was repeated. The mixture was then cooled to 90°C, a stream of demineralized water was added and the mixture was then stirred. The water was removed by vacuum distillation.
Molecular weight: Mn = 7500 g/mol, Mw = 19 000, Mw/Mn = 2.6
Melting point: 49°C

### Formulation examples 1 to 3

The following three formulations 1, 2 and 3, for which formulations the stability of the compositions and the photostability of compound (2) of the invention were evaluated, were prepared.

| | Ex 1 | | Ex 2 | | Ex 3 | |
|---|---|---|---|---|---|---|
| | According to the invention | | Outside the invention | | Outside the invention | |
| Disodium EDTA | | 0.1 | | 0.1 | | 0.1 |
| Triethanolamine | | 1.19 | | 1.2 | | 1.2 |
| Phenoxyethanol | | 0.7 | | 0.7 | | 0.7 |
| Isopropyl lauroyl sarcosinate | | 15 | | 15 | | 15 |
| Butyl methoxydibenzoylmethane | | 1.5 | | 1.5 | | 1.5 |
| Titanium dioxide | | 3 | | 3 | | 3 |
| Terephthalylidenedicamphorsulfonic acid | | 6 | | 6 | | 6 |
| Octocrylene | | 7 | | 7 | | 7 |
| Drometrizole trisiloxane | | 4 | | 4 | | 4 |
| Bis-Ethylhexyloxyphenol methoxyphenyl triazine | | 1.5 | | 1.5 | | 1.5 |
| Diethylamino hydroxybenzoyl hexyl benzoate | | 2 | | 2 | | 2 |
| Methoxypropylamino cyclohexenylidene ethoxyethylcyanoacetate | | 3 | | 3 | | 3 |
| Xanthan gum | | 0.2 | | 0.2 | | 0.2 |
| Acrylates copolymer (Aqua SF1) | | 1.3 | | 1.3 | | 1.3 |
| Poly C10-30 alkyl acrylate (Intelimer IP13-1) | | | | | | 3 |
| Ammonium acryloyldimethyltaurate/VP copolymer | | 0.3 | | 0.3 | | 0.3 |
| Polymer 1 | | 3 | | | | |
| Polymethylsilsesquioxane | | 1 | | 1 | | 1 |
| Dimethicone (and) dimethiconol (Xiameter PMX-1503 Fluid) | | 1 | | 1 | | 1 |
| Vinyl Dimethicone/Methicone Silsesquioxane Crosspolymer | | 1 | | 1 | | 1 |
| PEG-8 | | 5 | | 5 | | 5 |
| Denatured alcohol | | 10 | | 10 | | 10 |
| Glycerol | | 8 | | 8 | | 8 |
| Caprylyl glycol | | 0.5 | | 0.5 | | 0.5 |
| Stearic acid | | | | 2 | | |
| Glyceryl stearate (and) PEG-100 stearate | | | | 2 | | |
| Potassium cetyl phosphate | | | | 1 | | |
| Sodium cocoyl sarcosinate | | | | 1 | | |
| Deionized water (qs) | | 100 | | 100 | | 100 |
| | | | | | | |
| Stability at 2 months, at T° ambient and at 45°C | Stable | | unstable | | unstable | |

### Emulsion preparation method:

The aqueous phase is heated at 90°C and homogenized using a magnetic bar. The Aqua SF1 polymer is subsequently incorporated, and is neutralized using triethanolamine with magnetic stirring. The aqueous filter is added to this phase while maintaining the temperature at 90°C and with stirring.

In parallel, the fatty phase is also prepared at 90°C with magnetic stirring. The fatty phase contains the surfactants, the liposoluble screening agents and the preservatives.

The emulsion is prepared by introducing the fatty phase into the aqueous phase at 3500 rpm using a VMI emulsifier of rotor/stator type (model: 4-blade rotor + stator grid) for 10 minutes. The mixture is cooled to 30°C. After cooling, the gelling agents are introduced and the emulsion is homogenized using a deflocculating device, then the fillers and the alcohol are introduced.

### Protocol for evaluating the screening efficiency

The sun protection factor (SPF) is determined according to the "in vitro" method described by B. L. Diffey in J. Soc. Cosmet. Chem. 40, 127-133, (1989). The measurements were made using a UV-2000S spectrophotometer from the company Labsphere. Each composition is applied to a rough plate of PMMA, in the form of a homogeneous and even deposit at a rate of 1 mg/cm².

### Protocol for evaluating the stability of the compositions of the invention

The stability of the compositions of the invention is evaluated by microscopic observations of their appearance. A composition is judged to be stable when the microscopic appearance and the viscosity are stable at T0 and for 2 months at ambient temperature, at 4°C and at 45°C.

### Protocol for evaluating the Photostability of the compositions of the invention

The photostability is evaluated by analytical assaying of the merocyanine in the formula before and after exposure to the equivalent of 2 h UVA. This corresponds to 100 min of exposure time under a UVA flux of 7.19× 10⁻³ W/cm².

### Results:

| | Ex 1 | Ex 2 | Ex 3 |
|---|---|---|---|
| | According to the invention | Outside the invention | Outside the invention |
| SPF (vitro) | 340 | 168 | 142 |
| PPD (vitro) | 521 | 218 | 186 |
| Photostability | 86% | 84% | 80% |

The composition according to the invention is stable and has a screening efficiency which is clearly greater than that of the compositions outside the invention.

The same type of result is obtained using polymer 2 according to the invention.

## Claims

1. Cosmetic or dermatological composition in the emulsion form, comprising in a physiologically acceptable support:
a) at least one aqueous phase
b) at least one oily phase
c) at least one merocyanine compound of formula (1) or one of the E/E- or E/Z-geometrical isomer forms thereof: 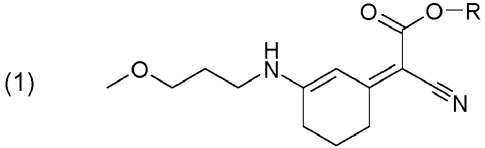 in which
R is a C₁-C₂₂ alkyl group, a C₂-C₂₂ alkenyl group, a C₂-C₂₂ alkynyl group, a C₃-C₂₂ cycloalkyl group or a C₃-C₂₂ cycloalkenyl group, said groups possibly being interrupted with one or more O, and
d) at least one polymer comprising monomeric units of formulae (A) and (B): 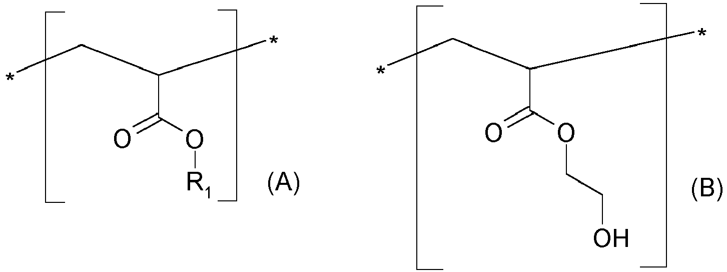 in which:
R₁, independently of one another, is chosen from alkyl or alkylene radicals;
and
at least 60% by weight of the R₁ groups are radicals chosen from stearyl and behenyl radicals, the percentage by weight relating to the sum of all the R₁ groups present in the polymer;
and
the weight ratio of the sum of all the hydroxyethyl acrylate units to the sum of all the acrylate units bearing the R₁ group ranges from 1: 30 to 1: 1;
and the sum of the total of units A and B is at least 95% by weight of the total weight of the polymer,
the polymer having a number-average molecular weight Mn ranging from 2000 to 9000 g/mol.

2. Composition according to Claim 1, wherein the merocyanine compound(s) are chosen from those wherein R is a C₁-C₂₂ alkyl which may be interrupted with one or more O.

3. Composition according to Claim 1 or 2, wherein the merocyanine compound(s) are chosen from the following compounds and also the E/E- or E/Z- geometrical isomer forms thereof:
| | | | |
|---|---|---|---|
| 1 | 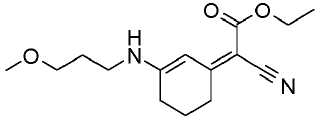 ethyl (2Z)-cyano{3-[(3-methoxypropyl)amino]cyclohex-2-en-1-ylidene}ethanoate | 4 | 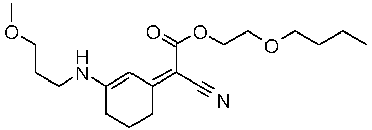 2-butoxyethyl (2Z)-cyano{3-[(3-methoxypropyl)amino]cyclohex-2-en-1-ylidene}ethanoate |
| 2 | 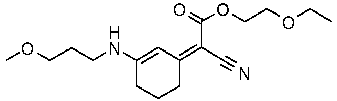 2-ethoxyethyl (2Z)-cyano{3-[(3-methoxypropyl)amino]cyclohex-2-en-1-ylidene}ethanoate | 5 | 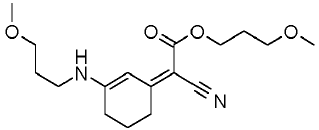 3-methoxypropyl (2Z)-cyano{3-[(3-methoxypropyl)amino]cyclohex-2-en-1-ylidene}ethanoate |
| 3 | 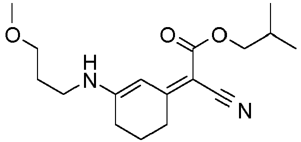 2-methylpropyl (2Z)-cyano{3-[(3-methoxypropyl)amino]cyclohex-2-en-1-ylidene}ethanoate | 6 | 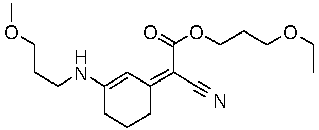 3-ethoxypropyl (2Z)-cyano{3-[(3-methoxypropyl)amino]cyclohex-2-en-1-ylidene}ethanoate |

4. Composition according to Claim 3, wherein the merocyanine compound is 2-ethoxyethyl (2Z)-cyano{3-[(3-methoxypropyl)amino]cyclohex-2-en-1-ylidene}ethanoate (2) in its E/Z geometrical configuration having the following structure: 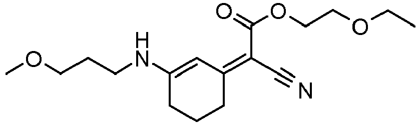 and/or in its E/E geometrical configuration having the following structure:

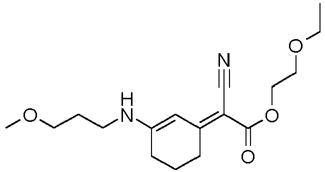

5. Composition according to any one of Claims 1 to 4, wherein the merocyanine(s) of formula (1) are present in a concentration ranging from 0.1% to 10% by weight, and preferentially from 0.2% to 5% by weight relative to the total weight of the composition.

6. Composition according to any one of Claims 1 to 5, **characterized in that**, in the polymer d), R₁ is constituted of an alkyl radical, preferably of a C₁₆-C₂₂ alkyl radical, and more preferentially of a behenyl or stearyl radical.

7. Composition according to any one of the preceding claims, **characterized in that**, in the polymer d), at least 70% by weight of the R₁ groups are behenyl or stearyl radicals, preferentially at least 80% by weight, more preferentially at least 90% by weight.

8. Composition according to any one of the preceding claims, in which, in the polymer d), all the R₁ groups are stearyl or behenyl radicals.

9. Composition according to any one of the preceding claims, **characterized in that**, in the polymer d), said weight ratio of the sum of all the hydroxyethyl acrylate units to the sum of all the acrylate units bearing the R₁ group ranges from 1:15 to 1:1, preferentially ranges from 1:10 to 1:4.

10. Composition according to any one of the preceding claims, **characterized in that** the polymer d) has a number-average molecular weight Mn ranging from 5000 to 9000 g/mol.

11. Composition according to any one of the preceding claims, in which the polymer d) has a melting point ranging from 40°C to 70°C, and preferentially ranging from 45°C to 67°C.

12. Composition according to any one of the preceding claims, **characterized in that**, in the polymer d), at least 60% by weight of the R₁ groups are stearyl radicals, and the polymer d) has a melting point ranging from 40 to 60°C, and preferentially ranging from 45 to 55°C.

13. Composition according to any one of Claims 1 to 11, **characterized in that**, in the polymer d), at least 60% by weight of the R₁ groups are behenyl radicals, and said polymer d) has a melting point ranging from 60°C to 70°C, and preferentially ranging from 63°C to 67°C.

14. Composition according to any one of the preceding claims, **characterized in that** the polymer(s) d) according to the invention are present in a content of active material ranging from 0.05% to 10% by weight, preferably ranging from 0.1% to 5% by weight and better still ranging from 0.2% to 2% by weight, relative to the total weight of the composition.

15. Composition according to any one of the preceding claims, wherein the composition is in the form of an oil-in-water emulsion.

## Patentansprüche

1. Kosmetische oder dermatologische Zusammensetzung in Emulsionsform, umfassend in einem physiologisch unbedenklichen Träger:
a) mindestens eine wässrige Phase
b) mindestens eine ölige Phase
c) mindestens eine Merocyanin-Verbindung der Formel (1) oder eine der geometrischen E/E- oder E/Z-Isomerformen davon: 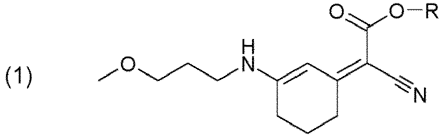 wobei:
R für eine C₁-C₂₂-Alkylgruppe, eine C₂-C₂₂-Alkenylgruppe, eine C₂-C₂₂-Alkinylgruppe, eine C₃-C₂₂-Cycloalkylgruppe oder eine C₃-C₂₂-Cycloalkenyl-gruppe steht, wobei die Gruppen gegebenenfalls durch ein oder mehrere 0 unterbrochen sein können, und
d) mindestens ein Polymer, das Monomereinheiten der Formeln (A) und (B) umfasst: 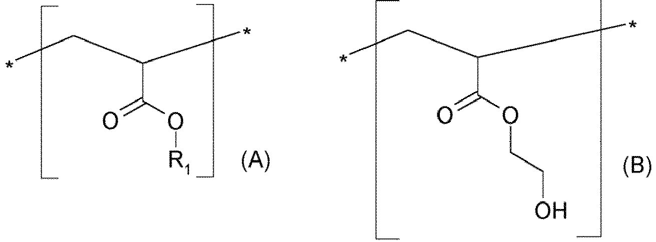 wobei:
R₁ unabhängig voneinander aus Alkyl- oder Alkylenresten ausgewählt ist
und
es sich bei mindestens 60 Gew.-% der R₁-Gruppen um Reste handelt, die aus Stearyl- und Behenylresten ausgewählt sind, wobei sich der Gewichtsprozentanteil auf die Summe aller in dem Polymer vorliegenden R₁-Gruppen bezieht;
und
das Gewichtsverhältnis der Summe aller der Hydroxyethylacrylat-Einheiten zu der Summe aller der Acrylat-Einheiten, die die R₁-Gruppen tragen, im Bereich von 1:30 bis 1:1 liegt
und die Summe der Gesamtheit von Einheiten A und B mindestens 95 Gew.-% des Gesamtgewichts des Polymers beträgt,
wobei das Polymer ein zahlenmittleres Molekulargewicht Mn im Bereich von 2000 bis 9000 g/mol aufweist.

2. Zusammensetzung nach Anspruch 1, wobei die Merocyanin-Verbindung(en) aus denjenigen ausgewählt sind, in denen R für ein C₁-C₂₂-Alkyl, das durch ein oder mehrere 0 unterbrochen sein kann, steht.

3. Zusammensetzung nach Anspruch 1 oder 2, wobei die Merocyanin-Verbindung(en) aus den folgenden Verbindungen sowie den geometrischen E/E- oder E/Z-Isomerformen davon ausgewählt sind:
| | | | |
|---|---|---|---|
| 1 | 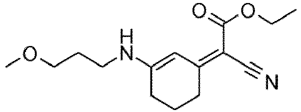 (2Z)-Cyano{3-[(3-methoxypropyl)amino]-cyclohex-2-en-1-yliden}ethansäure-ethylester | 4 | 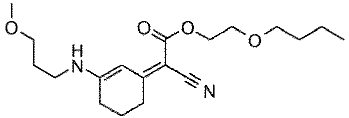 (2Z)-Cyano{3-[(3-methoxypropyl)amino]-cyclohex-2-en-1-yliden}ethansäure-2-butoxyethylester |
| 2 | 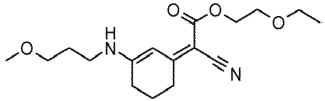 (2Z)-Cyano{3-[(3-methoxypropyl)amino]-cyclohex-2-en-1-yliden}ethansäure-2-ethoxyethylester | 5 | 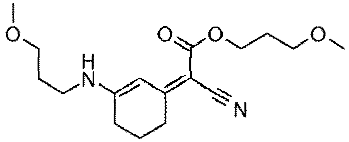 (2Z)-Cyano{3-[(3-methoxypropyl)amino]-cyclohex-2-en-1-yliden}ethansäure-3-methoxypropylester |
| 3 | 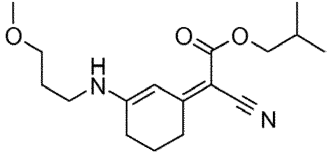 (2Z)-Cyano{3-[(3-methoxypropyl)amino]-cyclohex-2-en-1-yliden}ethansäure-2-methylpropylester | 6 | 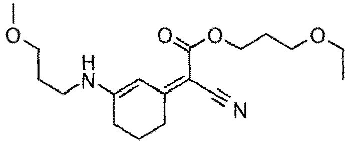 (2Z)-Cyano{3-[(3-methoxypropyl)amino]-cyclohex-2-en-1-yliden}ethansäure-3-ethoxypropylester |

4. Zusammensetzung nach Anspruch 3, wobei es sich bei der Merocyanin-Verbindung um (2Z)-Cyano{3-[(3-methoxypropyl)amino]cyclohex-2-en-1-yliden}ethan-säure-2-ethoxyethylester (2) in seiner geometrischen E/Z-Konfiguration mit der folgenden Struktur: 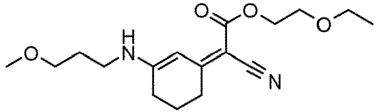 und/oder in seiner geometrischen E/E-Konfiguration mit der folgenden Struktur: 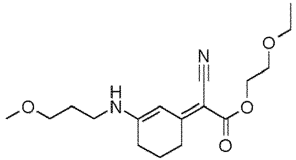 handelt.

5. Zusammensetzung nach einem der Ansprüche 1 bis 4, wobei die Merocyanin-Verbindung(en) der Formel (1) in einer Konzentration im Bereich von 0,1 bis 10 Gew.-% und vorzugsweise von 0,2 bis 5 Gew.-%, bezogen auf das Gesamtgewicht der Zusammensetzung, vorliegt bzw. vorliegen.

6. Zusammensetzung nach einem der Ansprüche 1 bis 5, **dadurch gekennzeichnet, dass** in dem Polymer d) R₁ aus einem Alkylrest, vorzugsweise aus einem C₁₆-C₂₂-Alkylrest und weiter bevorzugt aus einem Behenyl-oder Stearylrest besteht.

7. Zusammensetzung nach einem der vorhergehenden Ansprüche, **dadurch gekennzeichnet, dass** in dem Polymer d) mindestens 70 Gew.-% der R₁-Gruppen Behenyl- oder Stearylreste sind, bevorzugt mindestens 80 Gew.-%, weiter bevorzugt mindestens 90 Gew.-%.

8. Zusammensetzung nach einem der vorhergehenden Ansprüche, wobei in dem Polymer d) alle der R₁-Gruppen Stearyl- oder Behenylreste sind.

9. Zusammensetzung nach einem der vorhergehenden Ansprüche, **dadurch gekennzeichnet, dass** in dem Polymer d) das Gewichtsverhältnis der Summe aller der Hydroxyethylacrylat-Einheiten zu der Summe aller der Acrylat-Einheiten, die die R₁-Gruppe tragen, im Bereich von 1:15 bis 1:1 und bevorzugt im Bereich von 1:10 bis 1:4 liegt.

10. Zusammensetzung nach einem der vorhergehenden Ansprüche, **dadurch gekennzeichnet, dass** das Polymer d) ein zahlenmittleres Molekulargewicht Mn im Bereich von 5000 bis 9000 g/mol aufweist.

11. Zusammensetzung nach einem der vorhergehenden Ansprüche, in der das Polymer d) einen Schmelzpunkt im Bereich von 40 °C bis 70 °C und bevorzugt im Bereich von 45 °C bis 67 °C aufweist.

12. Zusammensetzung nach einem der vorhergehenden Ansprüche, **dadurch gekennzeichnet, dass** in dem Polymer d) mindestens 60 Gew.-% der R₁-Gruppen Stearylreste sind und das Polymer d) einen Schmelzpunkt im Bereich von 40 bis 60 °C und bevorzugt im Bereich von 45 bis 55 °C aufweist.

13. Zusammensetzung nach einem der Ansprüche 1 bis 11, **dadurch gekennzeichnet, dass** in dem Polymer d) mindestens 60 Gew.-% der R₁-Gruppen Behenylreste sind und das Polymer d) einen Schmelzpunkt im Bereich von 60 °C bis 70 °C und bevorzugt im Bereich von 63 °C bis 67 °C aufweist.

14. Zusammensetzung nach einem der vorhergehenden Ansprüche, **dadurch gekennzeichnet, dass** das Polymer bzw. die Polymere d) gemäß der Erfindung in einem Wirksubstanzgehalt im Bereich von 0,05 bis 10 Gew.-%, vorzugsweise im Bereich von 0,1 bis 5 Gew.-% und noch besser im Bereich von 0,2 bis 2 Gew.-%, bezogen auf das Gesamtgewicht der Zusammensetzung, vorliegt bzw. vorliegen.

15. Zusammensetzung nach einem der vorhergehenden Ansprüche, wobei die Zusammensetzung in Form einer Öl-in-Wasser-Emulsion vorliegt.

## Revendications

1. Composition cosmétique ou dermatologique sous la forme d'émulsion comprenant dans un support physiologiquement acceptable :
a) au moins une phase aqueuse
b) au moins une phase huileuse
c) au moins un composé de type mérocyanine de formule (1) ou l'une de ses formes géométriques isomères E/E ou E/Z 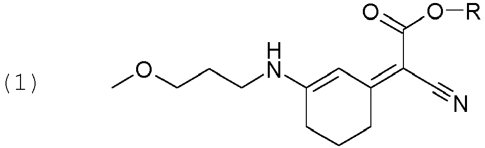 dans laquelle
R est un groupe alkyle en C₁-C₂₂, un groupe alcényle en C₂-C₂₂, un groupe alcynyle en C₂-C₂₂, un groupe cycloalkyle en C₃-C₂₂ ou un groupe cycloalcényle en C₃-C₂₂, lesdits groupes pouvant être interrompus par un ou plusieurs 0, et
d) au moins un polymère comprenant des unités monomériques de formules (A) et (B) : 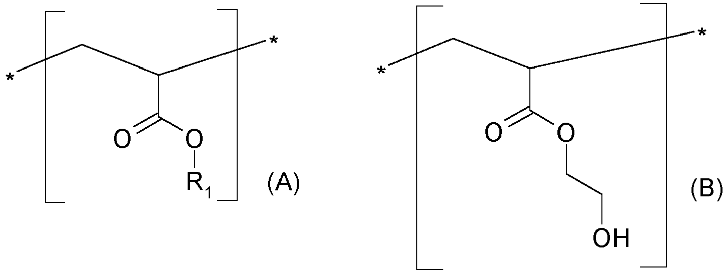 dans lesquelles :
R₁, indépendamment l'un de l'autre, est choisi parmi des radicaux alkyle ou alkylène,
et
au moins 60 % en poids des groupes R₁ sont des radicaux choisis parmi les radicaux stéaryle et béhényle, le pourcentage en poids se rapportant à la somme de tous les groupes R₁ présents dans le polymère,
et
le ratio pondéral de la somme de toutes les unités acrylate d'hydroxyéthyle sur la somme de toutes les unités acrylates portant le groupe R₁ va de 1 :30 à 1 : 1 ;
et la somme du total des unités A et B est d'au moins 95 % en poids du poids total du polymère,
le polymère ayant un poids moléculaire moyen en nombre Mn allant de 2 000 à 9 000 g/mol.

2. Composition selon la revendication 1, dans laquelle le ou les composés de type mérocyanine sont choisis parmi ceux dans lesquels R est un alkyle en C₁-C₂₂ qui peut être interrompu par un ou plusieurs 0.

3. Composition selon la revendication 1 ou 2, dans laquelle le ou les composés de type mérocyanine sont choisis parmi les composés suivants et également leurs formes géométriques isomères E/E ou E/Z :
| | | | |
|---|---|---|---|
| 1 | 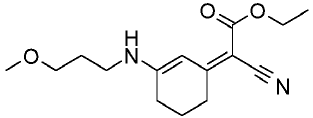 (2Z)-cyano{3-[(3-méthoxypropyl)amino]cyc lohex-2-en-1-ylidène}éthanoate d'éthyle | 4 | 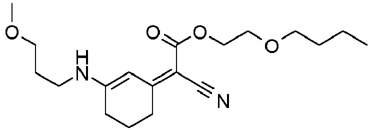 (2Z)-cyano{3-[(3-méthoxypropyl)amino]cyc lohex-2-en-1-ylidène}éthanoate de 2-butoxyéthyle |
| 2 | 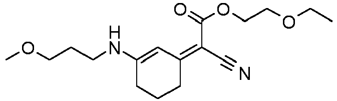 (2Z)-cyano{3-[(3-méthoxypropyl)amino]cyc lohex-2-en-1-ylidène}éthanoate de 2-éthoxyéthyle | 5 | 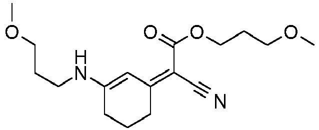 (2Z)-cyano{3-[(3-méthoxypropyl)amino]cyc lohex-2-en-1-ylidène}éthanoate de 3-méthoxypropyle |
| 3 | 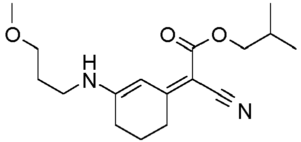 (2Z)-cyano{3-[(3-méthoxypropyl)amino]cyc lohex-2-en-1-ylidène}éthanoate de 2-méthylpropyle | 6 | 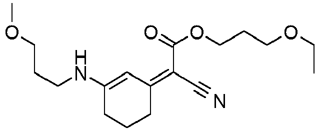 (2Z)-cyano{3-[(3-méthoxypropyl)amino]cyc lohex-2-en-1-ylidène}éthanoate de 3-éthoxypropyle. |

4. Composition selon la revendication 3, dans laquelle le composé de type mérocyanine est le (2Z)-cyano{3-[(3-méthoxypropyl)-amino]cyclohex-2-èn-1-ylidène}éthanoate de 2-éthoxyéthyle (2) dans sa configuration géométrique E/Z ayant la structure suivante : 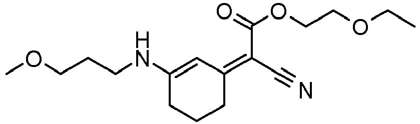 et/ou dans sa configuration géométrique E/E ayant la structure suivante :

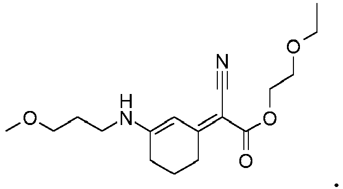

5. Composition selon l'une quelconque des revendications 1 à 4, dans laquelle la ou les mérocyanines de formule (1) sont présentes en une concentration allant de 0,1 % à 10 % en poids, et préférentiellement de 0,2 % à 5 % en poids par rapport au poids total de la composition.

6. Composition selon l'une quelconque des revendications 1 à 5, **caractérisée en ce que** dans le polymère d), R₁ est constitué par un radical alkyle, de préférence par un radical alkyle en C₁₆-C₂₂, et plus préférentiellement par un radical béhényle ou stéaryle.

7. Composition selon l'une quelconque des revendications précédentes **caractérisée en ce que** dans le polymère d) au moins 70 % en poids des groupes R₁ sont des radicaux béhényle ou stéaryle, de préférentiellement au moins 80 % en poids, plus préférentiellement au moins 90 % en poids.

8. Composition selon l'une quelconque des revendications précédentes dans laquelle dans le polymère d) tous les groupes R₁ sont des radicaux stéaryle ou béhényle.

9. Composition selon l'une quelconque des revendications précédentes **caractérisée en ce que** dans le polymère d), ledit ratio pondéral de la somme de toutes les unités acrylate d'hydroxyéthyle sur la somme de toutes les unités acrylate portant le groupe R₁ va de 1 : 15 à 1 : 1, préférentiellement va de 1 : 10 à 1 : 4.

10. Composition selon l'une quelconque des revendications précédentes **caractérisée en ce que** le polymère d) a un poids moléculaire moyen en nombre Mn allant de 5 000 à 9 000 g/mol.

11. Composition selon l'une quelconque des revendications précédentes dans laquelle le polymère d) a un point de fusion allant de 40 °C à 70 °C, et préférentiellement allant de 45 °C à 67 °C.

12. Composition selon l'une quelconque des revendications précédentes **caractérisée en ce que** dans le polymère d) au moins 60 % en poids des groupes R₁ sont des radicaux stéaryle, et le polymère d) a un point de fusion allant de 40 à 60 °C, et préférentiellement allant de 45 à 55 °C.

13. Composition selon l'une quelconque des revendications 1 à 11 **caractérisée en ce que** dans le polymère d) au moins 60 % en poids des groupes R₁ sont des radicaux béhényle, et ledit polymère d) a un point de fusion allant de 60 °C à 70 °C, et préférentiellement allant de 63 °C à 67 °C.

14. Composition selon l'une quelconque des revendications précédentes, **caractérisée en ce que** le ou les polymères d) selon l'invention sont présents en une teneur en matière active allant de 0,05 % à 10 % en poids, de préférence allant de 0,1 % à 5 % en poids et mieux encore allant de 0,2 % à 2% en poids, par rapport au poids total de la composition.

15. Composition selon l'une quelconque des revendications précédentes, dans laquelle la composition est sous la forme d'une émulsion huile-dans-eau.
